# EUROPEAN PATENT SPECIFICATION

(11) **EP 0 420 460 B1**
(45) Date of publication and mention of the grant of the patent: **30.11.1994**
(21) Application number: 90310084.0
(22) Date of filing: 14.09.1990
(51) Int. Cl.: A61F 2/38

(54) **Knee prosthesis**
Kniegelenkprothese
Prothèse du genou

(30) Priority: 27.09.1989 GB 8921753
(43) Date of publication of application: 03.04.1991
(73) Proprietor: UNIVERSITY COLLEGE LONDON, London WC1E 6BT (GB)
(72) Inventor: May, Denis Ronald William, c/o Dept. Biomed., Hospital, Stanmore, Middlesex (GB)
(74) Representative: Beresford, Keith Denis Lewis

(56) References cited:
- WO-A-89/06947
- DE-A- 2 122 390
- DE-A- 2 539 717
- FR-A- 2 076 838
- FR-A- 2 330 377
- FR-A- 2 445 137
- FR-A- 2 628 316
- GB-A- 1 457 147
- US-A- 3 837 009
- US-A- 3 909 854

## Description

The present invention relates to a knee prosthesis for implanting into a leg.

Knee prostheses in which a femoral member and a tibial member are hinged together are known. One such prosthesis is the so-called Stanmore hinged knee (see Lettin A. W. F., Journal of Bone and Joint Surgery, 1978, 60-b, 327-332. Another such prosthesis is the Attenborough knee (see Attenborough C. G., Journal of Bone and Joint Surgery 1978, 60-b, 320-6). Although many patients have been treated successfully with these knees, further surgery has been required in a number of patients because the joints have loosened or become broken (see R. J. Grimer, Journal of Bone and Joint Surgery 1984, 66-b, 55 and C. J. Kershaw, Journal of Bone and Joint Surgery 1988, 70-b, 89).

FR-A-2445137 discloses a knee joint in which tibial and femoral members are hinged together, that permits knee twisting, and in which a cam system converts the twisting movement into movement axially apart. However, the cam surfaces which would normally be of metal on twisting make only small area metal-to-metal contact with one another, which promots wear thereof.

DE 2539717 discloses a knee prosthesis comprising a femoral member connected to a tibial member by an axle and lateral ball-joints. The axle holding the femeral member by engagement with a hole in the femoral member and the lateral ball-joints engaging with corresponding sockets on the tibial member. The prosthesis also has a sliding area formed on the femoral member which bears or slides on the tibial member as the knee joint moves.

According to the present invention there is provided a knee prosthesis as set out in claim 1. Optional features are set out in the remaining claims.

The above knee prosthesis provides a hinged knee replacement with provision for limited rotation at the knee so that twisting forces are less likely to be transmitted to the cemented joints between the prosthesis and the tibia and femur, but in which the tendency for the working parts to wear is reduced. In particular, in order to minimize wear at the hinge axle the tibial member has a support for condylar surfaces of the femoral member to produce a reaction to at least part and preferably to a major part of the weight on the femoral member. In a further feature, the prosthesis has an extended position defined by abutment of an anterior portion of the condylar surfaces with a support. If the support is a member of low-friction plastics material that is carried on and fits into the tibial member, metal to metal contact at the extended position of the knee is avoided.

In order to permit twisting of the knee, a hinge axle having a cylindrical outer surface fits into a pivot hole of the femoral member that has enlarged ends and is internally shaped so as to roll on the axle as it twists . The femoral member is positioned transversely of the knee by medial and lateral ball formations that fit between corresponding socket formations that are non-twistable relative to the tibial member . The axle is defined by a pin which is preferably metallic and by a pair of bushes that are preferably of plastics and fit onto the axle from opposite ends with end flanges of the bushes fitting between the femoral and tibial members. The socket surfaces are then formed on inner faces of the flanges.

The invention will now be further described, by way of example only, with reference to the accompanying drawings in which:
Figure 1 is a perspective view of a Stanmore knee in a partly flexed position;
Figure 2 is a view of a knee prosthesis according to the invention in a flexed position;
Figure 3 is a perspective exploded view of the knee prosthesis of Figure 2;
Figure 4 is a front view of the prosthesis when unflexed: and
Figure 5 is a view of the prosthesis in transverse section.

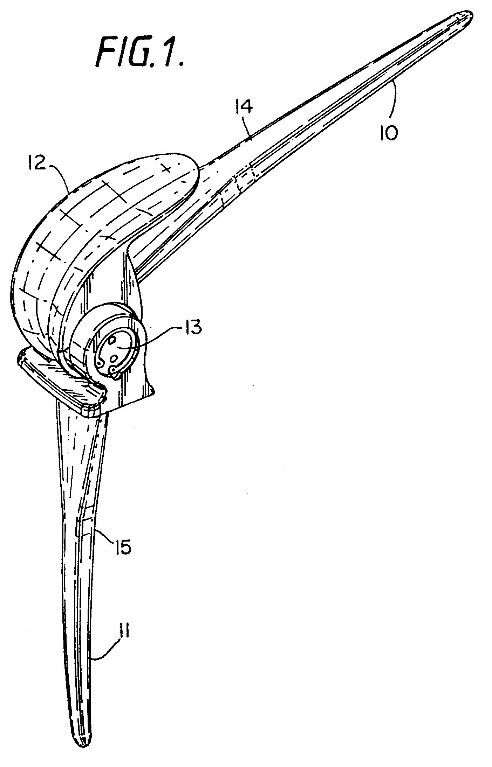

In the drawings, Figure 1 shows a Stanmore knee which has been implanted into over 10,000 patients since 1968. Further surgery in a minority of patients has been required because of loosening of fixation, fracture of one or other of the intramedullary stems 10, 11 and other causes. There are a number of other features of this knee which could be improved. The patella flange 12 is narrow and not very anatomic in profile and shape. This has resulted in subluxation and dislocation of the patella with consequential retropatella pain and discomfort. The hinge axis 13 is more anterior than the anatomic knee hinge position, resulting in a reduced lever arm of the patella and consequential extension lag and reduced quadriceps efficiency particularly when rising from a chair or when ascending stairs. The hinge is relatively narrow and high moments acting on it result in excessive wear of plastics axle bushes fitted into the knee which results in varus valgus instability. Particles which have become detached from the prosthesis as a result of wear have nowhere to escape and accelerate wear of the hinge surfaces. The hyperextension stop that defines the unflexed position of the knee occurs on abutment of anterior portions of the femoral and tibial components 14, 15 relatively compact areas, with direct metal to metal contact, further aggravating the debris from wear of the metal. The torsional rigidity of the Stanmore knee can not only cause loosening of the implant but in two cases known to the applicants has caused torsional fractures of the femur. However, the Stanmore knee is simple to manufacture and can be fitted to a patient by a relatively simple and quick surgical procedure which has accounted for its popularity notwithstanding the problems mentioned above.

An object of the invention is to provide a knee prosthesis for implantation into patients that is not significantly more complicated than the Stanmore knee and is surgically simple to fit but which does not suffer from the problems of that knee.

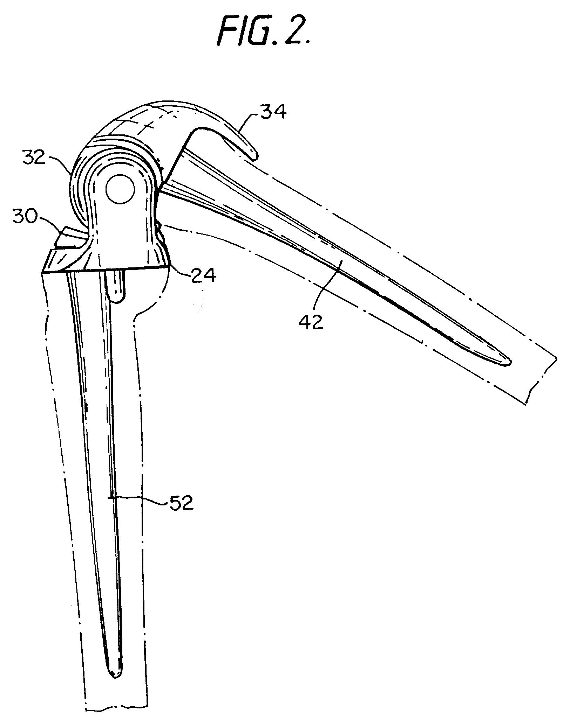

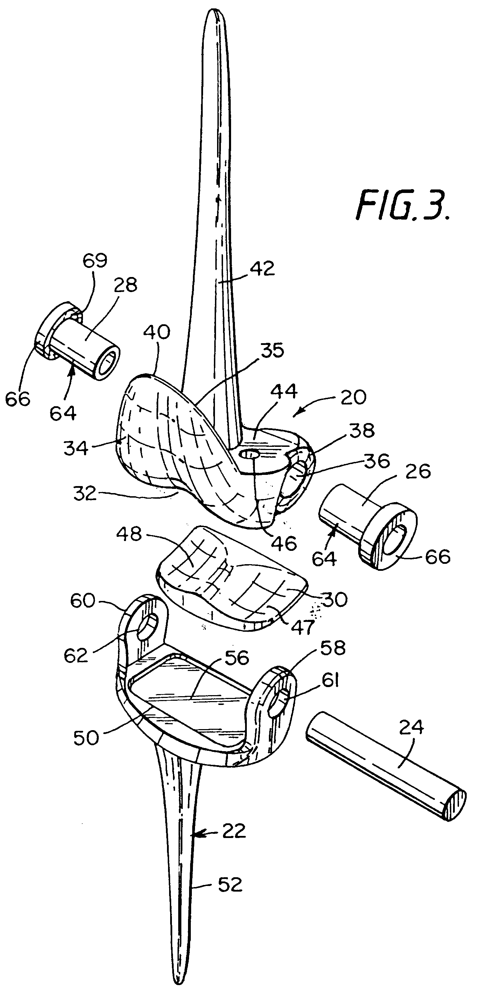

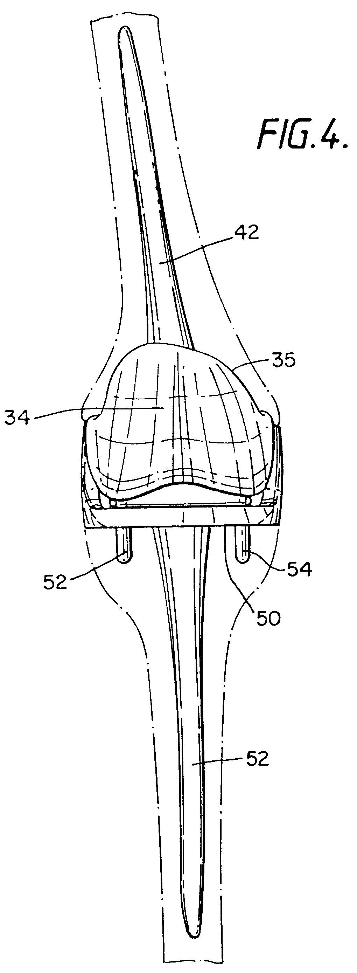

In Figures 2 to 5, a knee prosthesis comprises a femoral member 20 and a tibial member 22 which are each cast in a hard physiologically acceptable metal, for example cobalt chromium molybdenum alloy. The members 20, 22 are hingedly connected by a two-part axle consisting of an inner metallic axle pin 24 and a pair of axle bushes of plastics material 26, 28 that fit onto the axle pin 24. A tibial bearing member 30 of low-friction plastics material, for example ultra high molecular weight polyethylene. fits between the femoral and tibial members 20, 22.

The femoral member 20 has anatomically shaped and profiled condylar surfaces 32 leading to a broad patella flange 34 whose antero-proximal aspect 35 is shaped according to whether the knee is for the left or the right leg and which minimizes interference with the various blood vessels penetrating the bony cortex of the femur in the region of the patella. A transverse pivot hole 36 is located in or close to an anatomic position at the effective centre of rotation of the condylar surfaces 32, thus maintaining the natural lever arm of the patella and giving good quadriceps efficiency. The member 20 has convex spherical medial and lateral surfaces 38, 40 which locate and mate with corresponding concave seating surfaces 68, 69 of the bushes 26, 28 so that the femoral member rotates in and is located along a transverse direction by a ball joint. A femoral inter-medullary stem 42 tapered with four flats is set at a genu-vagum angle of 7° and arises from a femoral plateau 44 formed with lightening holes 46 that also act to reinforce the cement mantle and improve the torsional fixing.

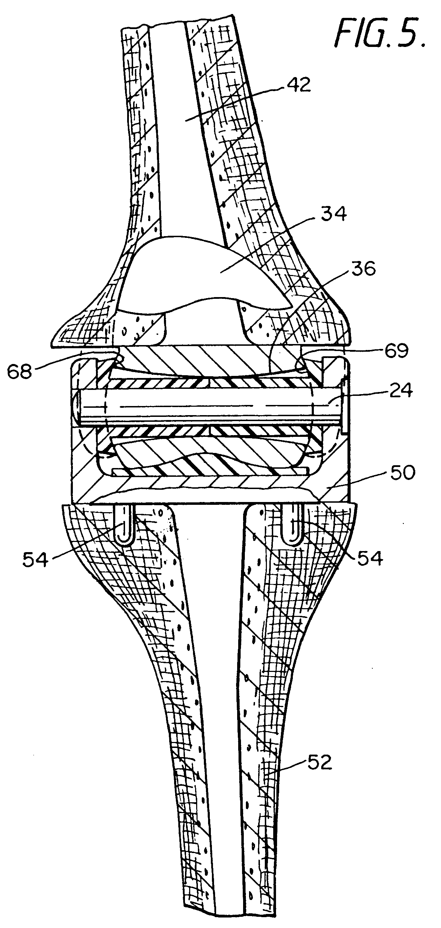

The tibial member 22 has a tibial tray 50 from the lower face of which a tibial intermedullary stem 52 depends at an angle of 3° of valgus. Because the stems 42, 52 are angled and the tray 50 is shaped to confirm to the top of the tibia , each component of the knee is left- or righthanded. A pair of anti-rotation pylons 54 spaced from the stem 52 depend from the tray 50 and are located so as to correspond to regions of greatest bone density. A recess 56 is formed in a proximal region of the tibial tray 50 and the tibial bearing member 30 is a snap fit into the recess 56. Medial and lateral lugs 58, 60 upstanding from the tray 50 are formed with apertures 61, 62 to receive the axle which is retained in position by a circlip (not shown). As is apparent from Figure 5, the tibial tray 50 is relatively large and when implanted extends over substantially the whole of the head of the tibia.

As previously stated, the axle is in two parts with the bushes 26, 28 that define the outer portion fitting loosely from opposite ends into the pivot hole 36 and abutting midway along the pivot hole. The concave socket surfaces 68, 69 locate on and slide freely over the medial and lateral surfaces 38, 40 of the femoral member 20. The member 20 carrying the bushes 26, 28 fits closely between the medial and lateral lugs 58, 60 of the tibial member 22, after which the axle pin can be passed through the apertures 61, 62 and the bushes 26, 28 and held in place by circlips to complete assembly of the joint. The bushes 26, 28 are twice as long as the corresponding bushes in the Stanmore hinge, which is expected to reduce the peak lateral contact stresses by a factor of 8.

In the Stanmore hinge the whole of the patient's weight is taken on the hinge bushes, whereas in the present knee a major proportion of the patient's weight is transmitted through the condylar surfaces 32 to the tibia. For this purpose, the tibial bearing member 30 is formed with dished bearing surfaces 47, 48 conforming to the curvature of the condylar surfaces 32 which allow for articulation of the members 20, 22 when the prosthesis is flexed and which provide support when the prosthesis is unflexed. In the unflexed position, an anterior portion of the condylar surfaces 32 abuts and anterior portion of the bearing member 30 to provide a hyperextension stop without metal to metal contact. Thus the condylar surfaces 32 slide over posterior regions of the member 30 when the knee is flexed and return when the knee is unflexed until the anterior portions of the condylar surfaces 30 and the member 32 abut to define the hyperextended or unflexed position.

The involute or reverse barrel shape of the pivot hole 36 permits torsional laxity of the hinge joint of up to 8°, the joint being controlled by the ball surfaces 38, 40 that fit into the socket surfaces 68, 69. It is expected that the loads on the bushes 26, 28 will be relatively light, and consequently that there will be few wear problems. The divergent or barrel form of the pivot hole 36 provides a route for egress of any particles that become detached as a result of wear, thus reducing acceleration of wear through retention of particles between the hinge surfaces.

The prosthesis described above provides controlled flexion and extension together with a high degree of medial-lateral stability for varus valgus movements. There is good conformity of the bearing surfaces which gives good kinematic location, and the relatively low contact stresses expected should give reduced wear and improved joint life. (It will be noted that the various bearing surfaces are spherical or involute which avoids high point contact stresses). The torsional articulation between the femoral member 20 and the bushes 26, 28 of the axle can allow internal-external rotation of typically up to about 8°. On twisting of the femur relative to the tibia, the condylar surfaces 32 ride up on the dished surfaces 47, 48 of the bearing member 30 and when the patient's weight is applied to the knee joint the axial load thereon provides a returning or centralizing torque which is similar to the screw-home axis of the knee described by some anatomists.

## Claims

1. A knee prosthesis comprising a femoral member (20) connected to a tibial member (22) by a hinge (24,61,62) that permits circumduction of the knee and prevents relative translational movement of the femoral and tibial members (20, 22) in a medial-lateral direction, an axle connecting the femoral and tibial members (20, 22) fitting into a pivot hole (36) of the femoral member (20) whose ends are larger than its central region and which is generally of reverse barrel shape, the tibial member (22) having a support member (30) for providing a reaction to at least part of a load which in use is transmitted through condylar surfaces (32) of the femoral member (20) and for preventing relative rotation of the tibial and femoral members (20, 22) in a medical-lateral plane, characterised in that the axle is defined by a pin (24) and a pair of bushes (26,28) that fit onto the pin and have end flanges (66) that fit between the femoral and tibial members (20,22) and wherein the femoral member is positioned transversely of the knee by medial and lateral ball surfaces (38) on the femoral member (20) that fit between socket surfaces (68,69) formed on said bushes (26,28) at the inner faces of said flanges.

2. A prosthesis according to Claim 1, wherein the support member (30) is formed with dished bearing surfaces (47,48) conforming to the curvature of the condylar surfaces (32) so that on twisting of the tibia] member (22) relative to the femoral member (20) the condylar surfaces (32) ride up on the dished surfaces (47,48) so that weight applied through the knee has an untwisting action.

3. A prosthesis according to Claim 1 or 2, having an extended position defined by abutment of an anterior portion of the condylar surface (32) with the support member (30).

4. A prosthesis according to Claim 3, wherein the support member (30) is a member of low-friction plastics material.

5. A prosthesis according to any preceding claim, wherein the pin (24) is of metal and the bushes (26,28) are of a plastics material.

## Patentansprüche

1. Kniegelenkprothese mit einem Femurelement (20), welches mit einem Tibiaelement (22) durch ein Gelenk (24, 61, 62) verbunden ist, welches Beugung (circumduction) des Knies gestattet und relative translatorische Bewegung der Femur- und Tibiaelemente (20, 22) in einer medial-lateralen Richtung verhindert, einer das Femur- und das Tibiaelement (20, 22) verbindenden Achse, eingepaßt in ein Zapfenloch (36) des Femurelements (20), dessen Enden größer als sein zentraler Abschnitt sind und welches generell eine umgekehrte Faßform hat, wobei das Tibiaelement (22) ein Lagerungselement (30) aufweist, um eine Umwandlung bzw. Gegenreaktion wenigstens Teils einer Last bzw. gegen einen Teil einer Last vorzusehen, welche bei Benutzung durch Gelenkkopfoberflächen (32) des Femurelements (20) übertragen wird, und um eine relative Drehung des Tibia- und Femurelementes (20, 22) in einer medial-lateralen Ebene zu unterbinden, dadurch gekennzeichnet, daß die Achse durch einen Stift (24) und einem Buchsenpaar (26, 28) definiert ist, welches auf dem Stift aufsitzt und Endscheiben (66) aufweist, die zwischen dem Femur- und dem Tibiaelement (20, 22) eingepaßt sind, und indem das Femurelement quer zum Knie durch mittlere und laterale Kugeloberflächen (38) des Femurelements (20) positioniert ist, welche zwischen Pfannenoberflächen (68, 69) eingepaßt sind, die auf den Buchsen (26, 28) an den inneren Flächen der Scheiben ausgebildet sind.

2. Prothese nach Anspruch 1, dadurch gekennzeichnet, daß das Stützelement (30) mit abgerundeten Stützoberflächen (47, 48) ausgebildet ist, welche der Wölbung der Gelenkkopfoberflächen (32) angepaßt sind, so daß beim Verdrehen des Tibiaelements (22) relativ zum Femurelement (20) die Gelenkkopfoberflächen (32) auf den abgerundeten Oberflächen (47, 48) hochrutschen, wobei ein auf das Knie wirkendes Gewicht keine verdrehende Wirkung ausübt.

3. Prothese nach Anspruch 1 oder 2, dadurch gekennzeichnet, daß eine ausgestreckte Stellung durch ein Anschlagen eines vorderen Abschnitts der Gelenkkopfoberfläche (32) mit dem Stützelement (30) definiert ist.

4. Prothese nach Anspruch 3, dadurch gekennzeichnet, daß das Stützelement (30) ein Element aus einem Plastikmaterial mit geringer Reibung ist.

5. Prothese nach einem der vorangegangenen Ansprüche, dadurch gekennzeichnet, daß der Stift (24) metallisch ist und die Buchsen (26, 28) aus Plastikmaterial sind.

## Revendications

1. Une prothèse de genoux comprenant une partie fémorale (20) reliée à une partie tibiale (22) par une charnière (24, 61, 62) qui permet une circonduction du genoux et empêche un mouvement de translation relatif des parties fémorales et tibiales (20, 22) dans une direction médiale-latérale, un axe reliant les parties fémorales et tibiales (20, 22) disposé dans un trou de rotation (36) de la partie fémorale (20) dont les extrémités sont plus grandes que sa région centrale et qui présente une forme générale de barreaux inversés, la partie tibiale (22) ayant un organe de support (30) permettant une réaction à au moins une partie d'une charge qui en fonctionnement est transmise par les surfaces condyliennes (32) de la partie fémorale (20), et empêchant une rotation relative des parties tibiales et fémorales (20, 22) dans un plan médial-latéral, caractérisée en ce que l'axe est défini par une tige (24) et une paire de manchons (26, 28) qui sont montées sur la tige et munies d'extrémité formant flasque (66) qui s'engagent entre les parties fémorales et tibiales (20, 22), et en ce que la partie fémorale est disposée transversalement aux genoux par des surfaces phériques médiales et latérales (38) de la partie fémorale (20) qui s'adapte entre les surfaces de butées (68, 69) formées sur lesdits manchons (26, 28) sur les faces internes desdits flasques.

2. Une prothèse selon la revendication 1, dans laquelle l'organe de support (30) est formé avec des surfaces d'appui en forme d'assiettes (47, 48) se conformant à la courbure des surfaces condylienne (32) de telle sorte qu'en cas de torsion de la partie tibiale (22) par rapport à la partie fémorale (20) les surfaces condyliennes glissent sur les surfaces en forme d'assiettes (47, 48) de telle sorte que la charge appliquée sur le genoux n'exerce pas d'effet de torsion.

3. une prothèse selon la revendication 1 ou 2, ayant une position en extension définie par butée d'une partie antérieure de la surface condylienne (32) avec l'organe de support (30).

4. Une prothèse selon la revendication 3, dans laquelle l'organe de support (30) est un organe en matériau plastique à faible coefficient de frottement.

5. Une prothèse selon l'une quelconque des revendications précédentes, dans laquelle la tige (24) est en métal et les manchons (26, 28) sont en matériau plastique.
